(19) Europäisches Patentamt
European Patent Office
Office européen des brevets

(11) **EP 3 279 666 A1**

(12) **EUROPEAN PATENT APPLICATION**

(43) Date of publication:
**07.02.2018 Bulletin 2018/06**

(51) Int Cl.:
***G01N 33/68*** *(2006.01)* ***B01D 59/26*** *(2006.01)*

(21) Application number: **16183106.0**

(22) Date of filing: **05.08.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **BASF Plant Science Company GmbH 67056 Ludwigshafen (DE)**

(72) Inventor: **The designation of the inventor has not yet been filed**

(74) Representative: **Herzog, Fiesser & Partner Patentanwälte PartG mbB Dudenstrasse 46 68167 Mannheim (DE)**

(54) **MEANS AND METHODS FOR DETERMINATION OF ISOTOPE LABELED AMINO ACIDS**

(57)   The present invention relates to a method for extracting at least one isotope-labelled compound from a complex matrix sample comprising a) contacting said complex matrix sample with an extraction solvent comprising a water-miscible organic solvent and water, b) binding cationic isotope-labelled compounds to a solid adsorbent in the presence of an acid, c) collecting non-cationic isotope-labelled compounds not binding to said solid adsorbent and/or eluting said cationic isotope-labelled compounds from said solid sorbent, d) thereby extracting at least one isotope-labelled compound from said complex matrix sample. The present invention further relates to a method for determining at least one isotope-labelled compound comprised in a complex matrix sample, comprising the aforesaid steps and the further steps of separating cationic and/or non-cationic isotope-labelled compounds by chromatographic steps, and determining, cationic and/or non-cationic isotope-labelled compounds. Further, the present invention relates to kits and uses related to said methods.

EP 3 279 666 A1

## Description

The present invention relates to a method for extracting at least one isotope-labelled compound from a complex matrix sample comprising a) contacting said complex matrix sample with an extraction solvent comprising a water-miscible organic solvent and water, b) binding cationic isotope-labelled compounds to a solid adsorbent in the presence of an acid, c) collecting non-cationic isotope-labelled compounds not binding to said solid adsorbent and/or eluting said cationic isotope-labelled compounds from said solid sorbent, d) thereby extracting at least one isotope-labelled compound from said complex matrix sample. The present invention further relates to a method for determining at least one isotope-labelled compound comprised in a complex matrix sample, comprising the aforesaid steps and the further steps of separating cationic and/or non-cationic isotope-labelled compounds by chromatographic steps, and determining, cationic and/or non-cationic isotope-labelled compounds. Further, the present invention relates to kits and uses related to said methods.

Methods for extracting biological compounds from biological samples, e.g. for quantification of metabolites contained in such sample, are known in the art. E.g. relating to protein amino acids, Heinzle et al.. (2008), Analytical Biochemistry, 380, 202-210 teach on analysis of C-13 labeling enrichment in microbial culture applying metabolic tracer experiments using gas chromatography-combustion-isotope ratio mass spectrometry. For analysis of free amino acids, Chen et al (2010), J. Chromatogr. B 878: 2199 describe microscale analysis of amino acids using gas chromatography-mass spectrometry after methyl chloroformate derivatization. Also, Kaspar et al. (2008), J. Chromatogr. B 870: 222 propose an automated GC-MS analysis of free amino acids in biological fluids. Further, Molero et al. (2014), Functional Plant Biology 41: 331-341 worked on the relationship between C and N fixation and amino acid synthesis in nodulated alfalfa (*Medicago sativa*), and proposed a method for measurement of $^{13}$C and $^{15}$N isotope labeling by gas chromatography-combustion-isotope ratio mass spectrometry to study amino acid fluxes in a plant-microbe symbiotic association (Molero et al. (2011), Rapid Commun. Mass Spectrom. 25: 599-607).

Over recent years, techniques of isotope ratio mass spectrometry (IRMS), in particular using $^{13}$C and $^{15}$N substrates, have gained interest, since they allow determination of fluxes of compounds within an organism. E.g. Larsen et al. (2009), Ecology, 90(12): 3526-3535 reported on stable isotope fingerprinting as a novel method for identifying plant, fungal, or bacterial origins of amino acids; and Dersch et al. (2016), Plant Physiology 171: 25-41 described a "Novel Approach for High-Throughput Metabolic Screening of Whole Plants by Stable Isotopes". Also, reviews on the topic of using IRMS in general and in plants in particular (cf. e.g. Brenna et al. (1997), Mass Spec Rev 16:227; Ghashghaie & Tcherkez (2013), "Isotope ratio mass spectrometry technique to follow plant metabolism" Book Chapter in Advances in Botanical Research, Volume 67, ISSN 0065-2296).

In IRMS, an analyte is combusted or otherwise thermally converted to defined molecules, which are then analyzed by mass spectrometry to detect mass differences between isotopes. This means that the actual analytes are no longer available in the actual quantification step. Thus, having the advantage of being sensitive, the technique of IRMS has the disadvantage that the actual quantification step does no longer allow identification of an analyte. Thus, IRMS is particularly sensitive to the quality of compound separation before detection.

There is, thus, a need in the art for improved methods for extracting compounds, in particular amino acids, from complex mixtures, avoiding at least in part the drawbacks of the prior art.

This problem is solved by the means and methods of the present invention, with the features of the independent claims. Preferred embodiments, which might be realized in an isolated fashion or in any arbitrary combination are listed in the dependent claims.

Accordingly, the present invention relates to a method for extracting at least one isotope-labelled compound from a complex matrix sample comprising

a) contacting said complex matrix sample with an extraction solvent comprising a water-miscible organic solvent and water,
b) binding cationic isotope-labelled compounds to a solid adsorbent in the presence of an acid,
c) collecting non-cationic isotope-labelled compounds not binding to said solid adsorbent and/or eluting said cationic isotope-labelled compounds from said solid sorbent,
d) thereby extracting at least one isotope-labelled compound from said complex matrix sample.

As used in the following, the terms "have", "comprise" or "include" or any arbitrary grammatical variation thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting further possibilities.

Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding further embodiments of the invention, without any restrictions regarding the scope of the invention and without any restriction regarding the possibility of combining the features introduced in such way with other optional or non-optional features of the invention. Moreover, if not otherwise indicated, the term "about" relates to the indicated value with the commonly accepted technical precision in the relevant field, preferably relates to the indicated value $\pm$ 20%, more preferably $\pm$ 10%, most preferably $\pm$ 5%.

The method for extracting of the present invention is an in vitro method. Moreover, it may comprise steps in addition to those explicitly mentioned above. For example, further steps may relate, e.g., to providing a sample for step a), or further separating, analyzing, or determining isotope-labelled compounds obtained in step c). Moreover, one or more of said steps may be performed by automated equipment.

The term "compound", as used herein, relates to a chemical molecule. Preferably, the term relates to at least one molecule of a specific compound up to a plurality of molecules of the said specific compound. It is to be understood further that a group of compounds, preferably, includes a plurality of chemically different molecules wherein for each compound at least one molecule up to a plurality of molecules may be present. Preferably, the compound is an inorganic chemical compound, more preferably an organic chemical compound. Preferably, a compound has a molecular weight of from 25 Da (Dalton) to 300,000 Da, more preferably of from 30 Da to 30,000 Da, most preferably of from 50 Da to 1500 Da. Preferably a metabolite has a molecular weight of less than 30,000 Da, less than 20,000 Da, less than 15,000 Da, less than 10,000 Da, less than 8,000 Da, less than 7,000 Da, less than 6,000 Da, less than 5,000 Da, less than 4,000 Da, less than 3,000 Da, less than 2,000 Da, less than 1,000 Da, less than 500 Da, less than 300 Da, less than 200 Da, or less than 100 Da. Preferably, a metabolite has, however, a molecular weight of at least 50 Da.

Preferably, the compound is a biological macromolecule, e.g. preferably, DNA, RNA, protein, or a fragment thereof, e.g., preferably a fragment produced by processing of sample material or by hydrolysis of the macromolecule. More preferably, in case a plurality of compounds is envisaged, said plurality of compounds is representing a metabolome, i.e. the collection of metabolites being comprised by an organism, an organ, a tissue, a body fluid, a cell or a part of a cell at a specific time and under specific conditions.

More preferably, the compound in accordance with the present invention is a small molecule compound, such as a substrate for an enzyme of a metabolic pathway, an intermediate of such a pathway or a product obtained by a metabolic pathway; thus, preferably, the compound is a metabolite. Metabolic pathways are well known in the art and may vary between species. Preferably, said pathways include at least citric acid cycle, respiratory chain, photo respiratory chain, glycolysis (Embden-Meyerhof-Parnas (EMP) pathway), gluconeogenesis, hexose monophosphate pathway, starch metabolism, oxidative and non oxidative pentose phosphate pathway (Calvin-Benson (CB) cycle), glyoxylate metabolism, production and $\beta$-oxidation of fatty acids, urea cycle, amino acid biosynthesis pathways, protein degradation pathways such as proteasomal degradation, amino acid degrading pathways, biosynthesis or degradation of lipids, polyketides (including e.g. flavonoids and isoflavonoids), isoprenoids (including e.g. terpenes, sterols, steroids, carotenoids, xanthophylls), carbohydrates, phenylpropanoids and derivatives, alcaloids, benzenoids, indoles, indole-sulfur compounds, porphyrines, anthocyans, hormones, vitamins, cofactors such as prosthetic groups or electron carriers, glucosinolates, purines, pyrimidines, nucleosides, nucleotides and related molecules. Accordingly, small molecule compounds are preferably comprised in at least one of the following classes of compounds: alcohols, alkanes, alkenes, alkines, aromatic compounds, ketones, aldehydes, carboxylic acids, esters, amines, imines, amides, cyanides, amino acids, small peptides, preferably of 2 - 10 amino acids, thiols, thioesters, phosphate esters, sulfate esters, thioethers, sulfoxides, ethers, or combinations or derivatives of the aforementioned compounds. The small molecules among the metabolites may be primary metabolites which are required for normal cellular function, organ function or animal or plant growth, development or health. Moreover, small molecule metabolites further comprise secondary metabolites having essential ecological function, e.g. metabolites which allow an organism to adapt to its environment. Furthermore, metabolites are not limited to said primary and secondary metabolites and further encompass artificial small molecule compounds. Said artificial small molecule compounds are derived from exogenously provided small molecules which are administered or taken up by an organism but are not primary or secondary metabolites as defined above, including, preferably, drugs, herbicides, fungicides, and insecticides. Moreover, artificial small molecule compounds may be metabolic products of compounds taken up, and preferably metabolized, by metabolic pathways of an organism. Moreover, small molecule compounds preferably include compounds produced by organisms living in, on or in close vicinity to an organism, more preferably by an infectious agent as specified elsewhere herein, by a parasitic and/or by a symbiotic organism.

As used herein, the term "analyte" refers to a molecular species determined according to the invention. Preferably, the analyte is a compound comprised in a sample. More preferably, the analyte is a metabolite itself which is found in a sample. Most preferably, the analyte is a molecular species which is derived from a compound or metabolite. In such a case, the actual metabolite will be chemically modified before and/or during the determination process and, as a result of said modification, a chemically different molecular species will be the determined molecular species. It is to be understood that in such a case, the analyte preferably represents the actual compound. Preferably, the amount of analyte is proportional to the amount of compound(s) the analyte is derived from.

Moreover, an analyte according to the present invention is not necessarily corresponding to one molecular species. Rather, the analyte may comprise stereoisomers or enantiomers of a compound. Further, an analyte can also represent the sum of isomers of a biological class of isomeric molecules, or of a subgroup thereof. Said isomers shall exhibit identical analytical characteristics in some cases and are, therefore, not distinguishable or distinguished by various analytical methods including those applied in the accompanying examples described below. However, preferably, the isomers will share at least identical sum formula and, thus, in the case of, e.g., lipids, an identical number of carbon atoms and identical numbers of double bonds in the sum of the fatty acid and other long-chain aliphatic moieties, e.g., sphingobase moieties. Depending on the method for determining at least one characteristic feature of an analyte chosen, an analyte may also represent a sum of a biological class of compounds, such as total amino acids, or a subgroup thereof, e.g. neutral amino acids; an analyte can also comprise isobars, represented by the same nominal mass but different sum formula; and/or an analyte may comprise molecules with a similar sub-structure, in particular in case the method for determining only detects said sub-structure or in case compounds are modified during processing, which may, e.g. include reactions such as losses of chemical groups or a molecular rearrangement occurring when chemical modification is carried out. Preferably, in IRMS, the analyte determined in determining isotopes of nitrogen is $N_2$, and/or the analyte determined in the determination of carbon isotopes is $CO_2$.

According to the present invention, at least one compound comprised in a complex matrix sample is an isotope-labelled compound. The term "isotope-labelled compound", as used herein, relates to a compound in which at least one atom was replaced by a non-identical isotope in at least a fraction of molecules of said compound. As is known to the skilled person, alternative isotopes may occur naturally; thus, preferably, an isotope-labelled compound is a compound in which the fractions of isotopes differ, preferably significantly, from the natural distribution. Preferably, a difference from the natural distribution is measured by IRMS; the method of IRMS and its applications, are, in principle, known to the skilled person, in particular from the literature cited herein in the introduction. More preferably, a difference from the natural distribution is measured by IRMS as specified herein in the Examples. Preferably, measuring deviation from the natural distribution comprises comparing the measured value to a reference value, wherein, more preferably, said reference value is obtained from a corresponding control sample. The term "corresponding control sample" is understood by the skilled person; preferably, the term relates to a sample treated with the least possible deviation from the sample of interest except the parameter to be analyzed. Thus, e.g. in isotope labeling, a sample treated identically but not being administered isotope label, or a sample withdrawn immediately before labelling is a corresponding control sample. Preferably, in an isotope-labelled compound at least one isotope selected from $^2H$, $^{13}C$, $^{15}N$, and $^{18}O$ differs from the unlabeled control sample. More preferably, the isotope-labelled compound is a $^{13}C$ labelled and/or $^{15}N$ labelled compound.

As is known to the skilled person, to describe the small variation of low level enrichment (minute variations in very small amounts of the heavier isotope are detected in the presence of large amounts of the lighter isotope) the $\delta$-notation in units of per mil [‰] has been adopted to report changes in isotopic abundance as a per mil deviation compared to a designated isotopic reference (Vienna Pee Dee Belemnite (VPDB) for determination of $\delta$-13C/12C, atmospheric nitrogen for $\delta$-15N/14N and Vienna Standard Mean Ocean Water (VSMOW) for

$$\delta = \left( \frac{R_{sample} - R_{standard}}{R_{standard}} \right) * 1000$$

$\delta$-$^2H/^1H$ and $\delta$-$^{18}O/^{16}O$, where R is the isotope ratio $^{13}C/^{12}C$, $^{15}N/^{14}N$, $^2H/^1H$ or $^{18}O/^{16}O$. $R_{sample}$ is the measured isotope ratio for the sample or the control sample and $R_{standard}$ is the measured isotope ratio for the designated isotopic reference. Preferably, in an isotope-labelled compound at least one isotope selected from $^2H$, $^{13}C$, $^{15}N$, and $^{18}O$ differs from the unlabeled control sample. Preferably, in an isotope-labelled compound, the isotope ratio of $^{13}C/^{12}C$ differs by at least 0.5‰, preferably at least 5‰, more preferably at least 10‰, most preferably at least 100‰. Also preferably, in an isotope-labelled compound, the isotope ratio of $^{15}N/^{14}N$ differs by at least 0.5‰, preferably at least 5‰, more preferably at least 10‰, most preferably at least 100‰. Also preferably, in an isotope-labelled compound, the isotope ratio of $^{18}O/^{16}O$ differs by a $\delta$-value of at least 1‰, preferably at least 5‰, more preferably at least 10‰, most preferably at least 100‰. Also preferably, in an isotope-labelled compound, the isotope ratio of $^2H/^1H$ differs by a $\delta$-value of at least 3‰, preferably at least 10‰, more preferably at least 50‰, most preferably at least 500‰.

Preferably, the isotope-labelled compound is a "cationic isotope-labelled compound", i.e. is an isotope-labelled compound having a positive net charge at pH = 1. Preferably, the cationic isotope-labelled compound is a compound having a positive net charge at pH = 1 and having no net charge or a negative charge at pH = 8. Thus, the cationic isotope-labelled compound preferably is a cationic compound under acidic conditions, preferably under the pH conditions of the extraction solvent. More preferably, the cationic isotope-labelled compound is a cationic compound under acidic conditions, preferably under the pH conditions of the extraction solvent, and is an anionic compound or a compound having no net charge under the conditions of the elution solvent a s specified elsewhere herein. As is understood by the skilled person, a compound having no net charge may also be a zwitterionic compound. Preferably the cationic isotope-labelled compound is a compound comprising at least one amino group, preferably a primary, secondary, or tertiary amino group, more preferably a primary amino group. Most preferably the cationic isotope-labelled compound is an amino acid, preferably a naturally occurring amino acid, more preferably an L-amino acid, most preferably a proteinogenic amino acid.

Also preferably, the isotope-labelled compound is a non-cationic isotope-labelled compound, i.e. is an isotope-labelled compound not having a positive net charge at pH = 1. Thus, preferably the non-cationic isotope-labelled compound is an anionic or neutral compound. Preferably, the non-cationic isotope-labelled compound is a neutral compound, more preferably a saccharide or an acidic compound. The saccharide, preferably, is a saccharide of the primary metabolism. The acidic compound, preferably, is an inorganic or, more preferably, organic acid compound, including, e.g. mono- and dicarbonic acids.

The term "matrix", as used herein, encompasses all constituents of a sample which are not compounds of interest. As will be understood by the skilled person, a sample, in particular a biological sample, may comprise hundreds or even thousands of species of chemical molecules, of which only a few or even only one may be of interest. Thus, in such case, the compounds which are not compounds of interest will collectively be referred to as matrix.

Accordingly, the term "complex matrix sample", as used herein, relates to a sample of a composition of matter comprising a complex matrix, i.e. comprising a multitude of compounds. Preferably, the complex matrix sample comprises at least 10, more preferably at least 20, even more preferably at least 50, most preferably at least 100 different compounds. Preferably, the complex matrix sample is a biological matrix sample, which is also referred to herein as biological sample. As used herein, the terms "biological matrix sample" and "biological sample" relate to a sample comprising, preferably consisting of, at least one biological material, preferably selected from a bodily fluid, a cell, a tissue, an organism, and an exudate. Preferably, a complex matrix sample is a sample from an archaebacterial, bacterial, or a eukaryotic cell.

More preferably, a complex matrix sample is a sample from a cultured cell, i.e., preferably, from a cell maintained ex vivo in an appropriate cell culture medium, preferably a cultured bacterial, plant, or mammalian cell, of a yeast, fungus, or algae, or a spent medium thereof. More preferably, the complex matrix sample is a sample from a subject, preferably a plant or animal subject. The plant subject, preferably, is a dicot or monocot plant, more preferably is a crop plant. The animal subject, preferably, is a mammalian subject, more preferably livestock or laboratory animal, most preferably a human subject. A bodily fluid sample, preferably, is a sample of stool, urine, saliva, cerebrospinal fluid, blood, serum, plasma, or lacrimal fluid. More preferably, the bodily fluid sample is a blood, serum, or a plasma sample.

As is understood by the skilled person, a sample may be stored, e.g. in a frozen state. Accordingly, a sample, preferably, is a thawed or a frozen sample. Preferably, the sample, in particular the biological sample, is a dried sample. Methods for drying samples are known in the art. More preferably, the sample is freeze-dried. Thus, preferably, the biological sample is a sample of freeze-dried fluid or freeze dried tissue material; preferably, the biological sample is a sample of freeze-dried plant material, preferably, freeze-dried leaf material, freeze-dried root material, freeze-dried shoot material, freeze-dried stem material and/or freeze-dried reproductive material, more preferably freeze-dried flower material, freeze-dried tassel, freeze-dried pollen material and/or freeze-dried seed material. The biological sample may also be a sample of homogenized biological material, e.g. ground tissue, preferably ground freeze-dried tissue, lysed cells, and the like.

As indicated above, the method of the present invention may comprise further steps. E.g., preferably, an internal standard may be added, before, upon, or after binding cationic isotope-labelled compounds to a solid adsorbent, preferably is added before said step. Preferably, in said internal standard up to four, more preferably up to two standard compounds, most preferably one standard compound are/is comprised. Preferably, the method includes treatments assisting in releasing or separating the analyte(s) or to remove excessive material or waste. Suitable techniques comprise centrifugation, extraction, fractioning, ultrafiltration, separation (e.g. by binding to paramagnetic beads and applying magnetic force), protein precipitation followed by filtration and/or centrifugation; and further purification and/or enrichment of compounds. Moreover, other pre-treatments are preferably carried out in order to provide the analyte(s) in a form or concentration suitable for compound analysis. For example, if gas-chromatography coupled mass spectrometry is used in the method of the present invention, it may be required to derivatize the analyte(s) prior to the gas chromatography.

Preferably, the pre-treatment of the sample allows for a subsequent separation of compounds, in particular of the small molecule compounds as referred to above, comprised by a sample. Molecules of interest, in particular the isotope-labelled compounds as referred to above may be extracted in an extraction step which comprises mixing of the sample with a suitable extraction solvent. The extraction solvent shall preferably be capable of precipitating macromolecules, in particular proteins and/or polynucleotides, in a sample, thereby facilitating the, preferably, centrifugation-based, removal of macromolecule contaminants which otherwise would interfere with the subsequent analysis of the analyte(s) as referred above. Preferably, at least the small molecule analyte(s) as referred to herein are soluble in the extraction solvent. Preferably, the extraction solvent mixture is a non-phase separating, i.e., a one-phase solvent mixture.

The term "extracting" as used herein, relates to transferring at least one compound of interest into an extraction solvent. Preferably, the term further relates to enriching at least one compound of interest over at least one further compound comprised in a sample.

The term "extraction solvent", as used herein, relates to a monophasic mixture comprising at least a water-miscible organic solvent and water. Preferably, the extraction solvent comprises further components, in particular an acid.

The term "water-miscible organic solvent" is, in principle, known to the skilled person and relates to an organic solvent which is, at least within a certain concentration range, miscible with water, the term miscible relating to the property of an organic solvent of being mixable with water without separating, i.e. preferably, without forming a two phase system. As is known to the skilled person, some organic solvents, e.g. ethanol and acetonitrile, are miscible with water at all ratios, whereas others are miscible only at specific concentrations; e.g. 2-butanol is miscible with water only up the 290 g/l. Thus, as used herein, the term water-miscible organic solvent includes organic solvents freely miscible with water, as well as organic solvents partially miscible with water at the concentrations where said organic solvent is miscible with water. Preferably, the organic solvent is selected from the list consisting of tetrahydrofuran, dimethylsulfoxide, acetonitrile, and an alcohol. More preferably, the water-miscible organic solvent is an alcohol, more preferably a monohydric alcohol. Preferably, said alcohol is a primary or secondary, preferably is a primary alcohol. Preferably, the water-miscible organic solvent is an alcohol comprising 4, 3, 2, or 1 carbon atom(s), preferably comprising 2 or 1 carbon atom(s), more preferably is methanol. Preferably, the water-miscible organic solvent, preferably said organic alcohol, more preferably methanol, is present in the extraction solvent at a concentration of from 10 % (v/v) to 95 % (v/v), preferably of from 25 % (v/v) to 90 % (v/v), more preferably of from 50 % (v/v) to 85 % (v/v), even more preferably of about 80 % (v/v), most preferably of 80 % (v/v). As is understood by the skilled person, the aforesaid concentration ranges are preferably adjusted to the solubility range of a given organic solvent.

The extraction solvent, preferably, further comprises an acid. Preferably, in such case, the presence of an acid during the step of binding cationic isotope-labelled compounds to a solid adsorbent is accomplished by applying the extraction solvent contacted with complex matrix sample directly, or, preferably, after removing insoluble compounds, to the solid adsorbent. Methods for removing insoluble compounds are known in the art and include, in particular, centrifugation and filtration methods. Preferably, removing insoluble compounds is the only step intervening the step of contacting the complex matrix sample with an extraction solvent and the step of binding cationic isotope-labelled compounds to a solid adsorbent.

The term "acid", as used herein, is known to the skilled person and, preferably, relates to an agent decreasing the pH of a solution. Thus, the acid, preferably, is a Brønsted acid. Preferably, the acid is a volatile acid, i.e. the acid can be evaporated, optionally under vacuum, without residue remaining. Also preferably, the extraction buffer comprising the acid is volatile. More preferably, the acid is an organic acid, most preferably an organic monoacid. Preferably the acid is a non-chlorinated and/or a non-fluorinated acid, more preferably a non-halogenated acid. Preferably, the acid is an organic acid comprising 4, 3, 2, or 1 carbon atom(s), preferably comprising 2 or 1 carbon atom(s), more preferably is formic acid. Preferably, the extraction solvent has a pH of at most 3; also preferably, the extraction solvent has a pH of from 1 to 3, more preferably of from 1.5 to 2.8, even more preferably of from 2 to 2.75, still more preferably of about 2.5, most preferably of 2.5. It is, however, also envisaged in case the complex matrix sample is strongly alkaline, that the pH of the extraction solution is lower than the aforesaid values; thus, preferably, the mixture resulting from contacting the complex matrix sample with the extraction solvent has a pH of at most 3; also preferably, said mixture has a pH of from 1 to 3, more preferably of from 1.5 to 2.8, even more preferably of from 2 to 2.75, still more preferably of about 2.5, most preferably of 2.5. Preferably, the acid is present in the extraction solvent at a concentration of at least 0.1 %. More preferably, the acid is present in said extraction solvent at a concentration of from 0.1 % (v/v) to 10 % (v/v), preferably of from 0.25 % (v/v) to 5 % (v/v), more preferably of from 0.5 % (v/v) to 2 % (v/v), even more preferably of about 1 % (v/v), most preferably of 1 % (v/v). As will be understood by the skilled person, extraction may, preferably, be performed in the absence of an acid, e.g. at neutral pH. Accordingly, an acid may be added to a sample after contacting said complex matrix sample with an extraction solvent, but before binding cationic isotope-labelled compounds to a solid adsorbent. Preferably, the acid , pH and/or concentration of said acid is as described herein above.

Preferably, the extraction solvent comprises, preferably consists of, methanol, water, and formic acid, preferably at a ratio of about 80/20/1 (v/v/v), more preferably of 80/20/1 (v/v/v).

According to the method of the present invention, non-cationic compounds, including isotope-labelled non-cationic compounds, not binding to said solid adsorbent are preferably collected. As will be understood by the skilled person, non-cationic isotope-labelled compounds are preferably collected by collecting, i.e. preferably combining, the fraction or fractions of compounds not binding to the solid adsorbent, preferably not binding to the solid cation adsorbent; thus, in case the solid adsorbent is packed in a column, e.g. the flow-through, and optionally the wash fraction, may be collected.

The term "solid adsorbent", as used herein, relates to a solid compound, preferably a particulate compound, comprising chemical groups imparting a net negative charge onto said solid compound, i.e. anionic groups. Thus, the solid adsorbent, preferably, is a solid cation adsorbent, more preferably a cation exchange material. Appropriate materials and chemical groups imparting a net negative charge are known to the skilled person. Preferred are chemical groups being anionic at a pH of 2.5, preferably at a pH of 1, the term "chemical groups being anionic at pH X" relating to a chemical group, wherein at least 50% of said chemical group are in a negatively charged form at pH of X. Thus, in case the said chemical group is an acid group, said acid group, preferably, has a $pK_A$ of at most 2.5. Preferably, said chemical groups are sulfonic acid groups. Preferably, the solid adsorbent has a pore size of at most 30 nm, more preferably at most 20 nm, even more preferably of 8 nm. Also preferably, the solid adsorbent has a pore size of from 1 nm to 30 nm, preferably of from 2 nm to 15 nm, more preferably of from 3 nm to 10 nm, even more preferably of about 8 nm.

Preferably, cationic compounds, preferably including cationic isotope-labelled compounds, are eluted from the solid adsorbent as specified herein above. Preferably, the solid adsorbent is washed before elution of cationic compounds, e.g., preferably, is washed with extraction solvent. Preferably, cationic compounds are eluted using an alkaline elution solution, more preferably a volatile alkaline elution solution. Preferably, the alkaline elution solution has a pH of at least 8, more preferably of at least 9. Preferably, the alkaline elution solution has a pH of from 8 to 14, more preferably of from 9 to 12. Preferably, according to the present invention, pH is measured according to IUPAC recommendations (Pure Appl. Chem. (2002), Vol. 74, No. 11, pp. 2169). Thus, preferably, pH measurements are performed at a temperature of 25°C. Preferably, the alkaline elution solution is a solution of $NH_4OH$, preferably at a concentration of from 0.1% to 25% (v/v), preferably of from 1% (v/v) to 10% (v/v), more preferably of about 5%(v/v), most preferably of 5% (v/v). Preferably, the alkaline elution solution is a solution of $NH_4OH$ at the aforesaid concentration in acetonitril.

Advantageously, it was found in the work underlying the present invention that, using the methods of the present invention, preparations of free amino acids and of neutral or acidic compounds which contain a particularly low amount confounding compounds can be obtained from complex mixtures such as biological samples. In particular, it was found that compounds purified according to the present invention permit a particularly good baseline separation in chromatography, e.g. in LC and, in particular, in GC applications. Moreover, since the method proposed contains a particularly low number of rebuffering, drying, and redissolving steps, it can be performed with a low amount of sample material.

The definitions made above apply mutatis mutandis to the following. Additional definitions and explanations made further below also apply for all embodiments described in this specification mutatis mutandis.

The present invention further relates to a method for determining at least one isotope-labelled compound comprised in a complex matrix sample, comprising

a) contacting said complex matrix sample with an extraction solvent comprising a water-miscible organic solvent and water
b) binding cationic isotope-labelled compounds to a solid adsorbent in the presence of an acid,
c) collecting non-cationic isotope-labelled compounds not binding to said solid adsorbent and/or eluting said cationic isotope-labelled compounds from said solid sorbent,
d) separating cationic isotope-labelled compounds by chromatographic steps and/or separating non-cationic isotope-labelled compounds by chromatographic steps, and
e) determining, preferably quantitatively determining, cationic and/or non-cationic isotope-labelled compounds, thereby
f) determining at least one isotope-labelled compound comprised in a complex matrix sample.

The method of the present invention, preferably, is an in vitro method. Moreover, it may comprise steps in addition to those explicitly mentioned above. For example, further steps may relate, e.g., to further purification and/or to derivatization steps. Moreover, one or more of said steps may be performed by automated equipment.

According to the present invention, determining cationic and/or non-cationic isotope-labelled compounds preferably comprises a non-compound discriminating determining step, preferably wherein said non-compound discriminating determining step is the only step of quantifying cationic and/or non-cationic isotope-labelled compounds.

Preferably, the step of separating cationic isotope-labelled compounds by chromatographic steps and/or separating non-cationic isotope-labelled compounds by chromatographic steps comprises liquid chromatography (LC) or gas chromatography (GC), preferably is GC. Methods of separating cationic and/or non-cationic compounds are known in the art and are, preferably, performed as specified herein in the Examples.

As used herein, the term "non-compound discriminating determining step" relates to a determining step comprising qualitative or, preferably quantitative determination of a compound. Thus, preferably, the non-compound discriminating determining step does not comprise a step of identifying a compound, e.g. by determining a characteristic feature of said compound. As will be understood by the skilled person, further steps of the present method may, preferably do, comprise a step of identifying a compound. Thus, e.g. a compound comprising $^{14}C$ may, e.g. be identified by its peak in an LC chromatogram, and the quantity of said compound may be determined by determining the amount of $^{14}C$ radioactivity in said peak. Preferably, the non-compound discriminating determining step comprises, preferably is, isotope ratio mass spectrometry (IRMS).

Thus, preferably, the step of determining, preferably quantitatively determining, cationic and/or non-cationic isotope-labelled compounds comprises, preferably consists of, combustion isotope ratio mass spectrometry (C-IRMS), preferably gas chromatography combustion isotope ratio mass spectrometry (GC-C-IRMS). Preferably, the IRMS is IRMS (IRMS) of $^{13}C$-comprising compounds, of $^{15}N$-comprising compounds, of $^{18}O$-comprising compounds, and/or of $^{2}H$-comprising compounds. More preferably, IRMS is thermal conversion IRMS (TC-IRMS) of $^{18}O$-comprising compounds and/or of $^{2}H$-comprising compounds. Most preferably, IRMS is combustion IRMS (C-IRMS) of $^{13}C$-comprising compounds and/or of $^{15}N$-comprising compounds.

Preferably, the method for determining at least one isotope-labelled compound comprises determining at least one cationic isotope-labelled compound and at least one non-cationic isotope-labelled compound.

The present invention further relates to a kit comprising an extraction solvent of the present invention and at least one isotope-labeled standard.

The term "kit", as used herein, refers to a collection of the aforementioned compounds, means or reagents of the present invention which may or may not be packaged together. The components of the kit may be comprised by separate housings (i.e. as a kit of separate parts) or may be provided in a single housing. Moreover, it is to be understood that the kit of the present invention, preferably, is to be used for practicing the methods referred to herein above. It is, preferably, envisaged that components, in an embodiment all components, are provided in a ready-to-use manner for practicing the methods referred to above. Preferably, all or some of said components are provided in dried, such as in lyophilized form, wherein the component is reconstituted using a liquid such as an aqueous buffered solution. Preferably, all or some of said components are provided in, preferably concentrated, more preferably ready-to-use, liquid form, wherein the concentrated component is diluted using a liquid such as an aqueous buffered solution. Further, the kit, preferably, contains instructions for carrying out said methods and, if applicable, said reconstitution of dried reagents. The instructions can be provided by a user's manual in paper form or electronic form. In addition, the manual may comprise instructions for interpreting the results obtained when carrying out the aforementioned methods using the kit of the present invention, and/or information on the value of the predetermined concentration of at least one analyte in the matrix calibration sample. Preferably, the kit further comprises at least one pre-treatment agent, a wash solution, and/or an elution solution. Preferably, the kit is a kit for use in IRMS, preferably for use in C-IRMS. Thus, preferably, the standard comprised in the kit comprises a $^{13}C$-enriched compound and/or a $^{15}N$-enriched compound. More preferably, the kit further comprises a solid adsorbent.

The present invention further relates to a use of a method according to the present invention in metabolic flux analysis.

The term "flux analysis" is understood by the skilled person and, preferably, relates to an analysis of the distribution and/or conversions of a compound and/or an element within a cell, tissue, or subject. Preferably, flux analysis is an analysis as described in WO 2014/079696.

The present invention also relates to a use of a solid adsorbent and/or of an extraction solvent comprising a water-miscible organic solvent and water in a method according to the present invention.

In view of the above, the following embodiments are preferred:

**Claims/Embodiments**

1. A method for extracting at least one isotope-labelled compound from a complex matrix sample comprising

   a) contacting said complex matrix sample with an extraction solvent comprising a water-miscible organic solvent and water
   b) binding cationic isotope-labelled compounds to a solid adsorbent in the presence of an acid,
   c) collecting non-cationic isotope-labelled compounds not binding to said solid adsorbent and/or eluting said cationic isotope-labelled compounds from said solid sorbent,
   d) thereby extracting at least one isotope-labelled compound from said complex matrix sample.

2. A method for determining at least one isotope-labelled compound comprised in a complex matrix sample, comprising

a) contacting said complex matrix sample with an extraction solvent comprising a water-miscible organic solvent and water
b) binding cationic isotope-labelled compounds to a solid adsorbent in the presence of an acid,
c) collecting non-cationic isotope-labelled compounds not binding to said solid adsorbent and/or eluting said cationic isotope-labelled compounds from said solid sorbent,
d) separating cationic and/or non-cationic isotope-labelled compounds by chromatographic steps, and
e) determining, preferably quantitatively determining, cationic and/or non-cationic isotope-labelled compounds, thereby
f) determining at least one isotope-labelled compound comprised in a complex matrix sample.

3. The method of embodiment 1 or 2, wherein said cationic isotope-labelled compound is an amino acid

4. The method of any one of embodiments 1 to 3, wherein said non-cationic isotope-labelled compound is a saccharide and/or an organic acid.

5. The method of any one of embodiments 1 to 4, wherein said water-miscible organic solvent is selected from the list consisting of tetrahydrofuran, dimethylsulfoxide, acetonitrile, and an alcohol.

6. The method of any one of embodiments 1 to 5, wherein said water-miscible organic solvent is an alcohol.

7. The method of any one of embodiments 1 to 6, wherein said water-miscible organic solvent is a monohydric alcohol.

8. The method of any one of embodiments 1 to 7, wherein said water-miscible organic solvent is a primary or secondary, preferably is a primary alcohol.

9. The method of any one of embodiments 1 to 8, wherein said water-miscible organic solvent is an alcohol comprising 4, 3, 2, or 1 carbon atom(s), preferably comprising 2 or 1 carbon atom(s), more preferably is methanol.

10. The method of any one of embodiments 1 to 9, wherein said water-miscible organic solvent, preferably said organic alcohol, is present at a concentration of from 10 % (v/v) to 95 % (v/v), preferably of from 25 % (v/v) to 90 % (v/v), more preferably of from 50 % (v/v) to 85 % (v/v), even more preferably of about 80 % (v/v), most preferably of 80 % (v/v).

11. The method of any one of embodiments 1 to 10, wherein said extraction solvent further comprises an acid.

12. The method of any one of embodiments 1 to 11, wherein said extraction solvent has a pH of at most 3, preferably of from 1 to 3, more preferably of about 2.5, most preferably of 2.5.

13. The method of any one of embodiments 1 to 12, wherein said acid is present in said extraction solvent at a concentration of at least 0.1%.

14. The method of any one of embodiments 1 to 13, wherein said acid is present in said extraction solvent at a concentration of from 0.1 % (v/v) to 10 % (v/v), preferably of from 0.25 % (v/v) to 5 % (v/v), more preferably of from 0.5 % (v/v) to 2 % (v/v), even more preferably of about 1 % (v/v), most preferably of 1 % (v/v).

15. The method of any one of embodiments 1 to 14, wherein said acid is a volatile acid.

16. The method of any one of embodiments 1 to 15, wherein said acid is an organic acid, preferably a non-halogenated acid.

17. The method of any one of embodiments 1 to 16, wherein said organic acid is an organic monoacid.

18. The method of any one of embodiments 1 to 17, wherein said acid is an organic acid comprising 4, 3, 2, or 1 carbon atom, preferably comprising 2 or 1 carbon atoms, more preferably is formic acid.

19. The method of any one of embodiments 1 to 18, wherein said extraction solvent comprises, preferably consists of, methanol, water, and formic acid, preferably at a ratio of about 80/20/1 (v/v/v), more preferably of 80/20/1 (v/v/v).

20. The method of any one of embodiments 1 to 19, wherein said cationic compounds are eluted from said solid sorbent by applying an alkaline elution solution to said solid adsorbent.

21. The method of any one of embodiments 1 to 20, wherein said alkaline elution solution is a volatile alkaline elution solution.

22. The method of any one of embodiments 1 to 20, wherein said alkaline elution solution is a solution of $NH_4OH$, preferably at a concentration of from 0.1% to 25%, preferably of from 1% to 10%, more preferably of about 5%, most preferably of 5%.

23. The method of any one of embodiments 1 to 22, wherein said complex matrix sample is a biological matrix sample.

24. The method of any one of embodiments 1 to 23, wherein said complex matrix sample comprises at least one of a bodily fluid, a cell, a tissue, an organism, and an exudate.

25. The method of any one of embodiments 1 to 24, wherein said cell is a mammalian cell, preferably a human cell, or is a plant cell, preferably a monocot or dicot cell.

26. The method of any one of embodiments 1 to 25, wherein said cell is a cultured cell.

27. The method of any one of embodiments 1 to 26, wherein said solid adsorbent is a solid cation adsorbent, preferably comprising anionic groups at a pH of 2.5.

28. The method of any one of embodiments 1 to 27, wherein said anionic groups comprise sulfonic acid groups, preferably wherein said anionic groups are sulfonic acid groups.

29. The method of any one of embodiments 1 to 28, wherein said solid adsorbent has a pore size of at most 30 nm, preferably at most 20 nm, more preferably of 8 nm.

30. The method of any one of embodiments 1 to 29, wherein said solid adsorbent has a pore size of from 1 nm to 30 nm, preferably of from 2 nm to 15 nm, more preferably of from 3 nm to 10 nm, even more preferably of about 8 nm.

31. The method of any one of embodiments 1 to 30, wherein said method further comprises a step of removing insoluble sample constituents before step b) and/or before step d).

32. The method of any one of embodiments 2 to 31, wherein said determining cationic and/or non-cationic isotope-labelled compounds comprises a non-compound discriminating determining step, preferably wherein said non-compound discriminating determining step is the only step of quantifying cationic and/or non-cationic isotope-labelled compounds.

33. The method of any one of embodiments 2 to 32, wherein said non-compound discriminating determining step is and/or comprises mass spectrometry.

34. The method of any one of embodiments 2 to 33, wherein said non-compound discriminating determining step comprises, preferably is, isotope ratio mass spectrometry (IRMS).

35. The method of any one of embodiments 2 to 34, wherein steps d) and e) comprise, preferably consists of, gas chromatography combustion isotope ratio mass spectrometry (GC-IRMS).

36. The method of any one of embodiments 34 to 35, wherein said IRMS is IRMS (IRMS) of $^{13}$C-comprising compounds, of $^{15}$N-comprising compounds, of $^{18}$O-comprising compounds, and/or of $^{2}$H-comprising compounds.

37. The method of any one of embodiments 34 to 36, wherein said IRMS is thermal conversion IRMS (TC-IRMS) of $^{18}$O-comprising compounds and/or of $^{2}$H-comprising compounds.

38. The method of any one of embodiments 34 to 36, wherein said IRMS is combustion (C-IRMS) of $^{13}$C-comprising compounds and/or of $^{15}$N-comprising compounds.

39. The method of any one of embodiments 2 to 38, wherein said chromatography is liquid chromatography (LC) or gas chromatography (GC), preferably is GC.

40. The method of any one of embodiments 2 to 39, wherein said method comprises determining at least one cationic isotope-labelled compound and at least one non-cationic isotope-labelled compound.

41. A kit comprising an extraction solvent as specified in any one of embodiments 1 to 40 and at least one isotope-labeled standard.

42. The kit of embodiment 41, wherein said standard comprises a $^{13}$C-enriched compound and/or a $^{15}$N-enriched compound.

43. The kit of embodiment 41 or 42, wherein said kit further comprises a solid adsorbent.

44. Use of a method according to any one of embodiments 1 to 40 in metabolic flux analysis.

45. Use of a solid adsorbent and/or of an extraction solvent comprising a water-miscible organic solvent and water in a method according to any one of embodiments 1 to 30.

46. The use of embodiment 45, wherein said extraction solvent further comprises an acid.

All references cited in this specification are herewith incorporated by reference with respect to their entire disclosure content and the disclosure content specifically mentioned in this specification.

Figure Legends

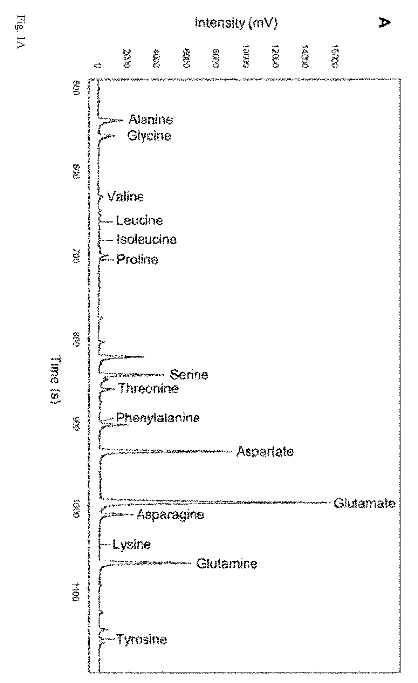

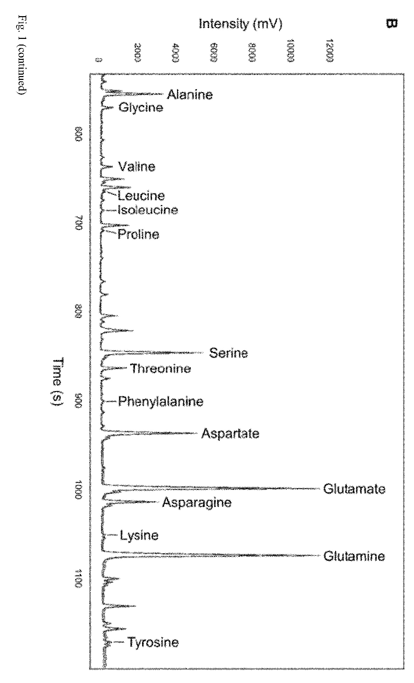

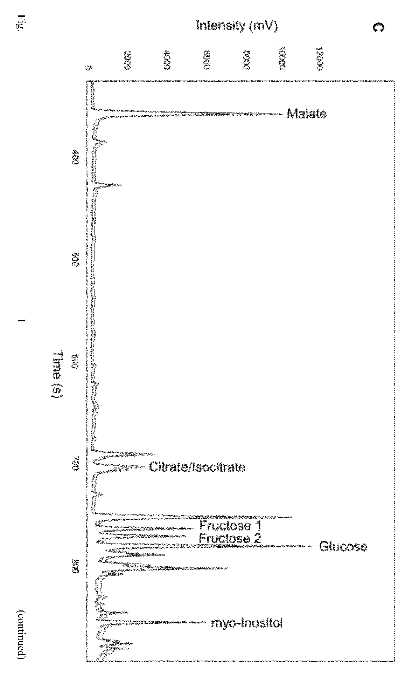

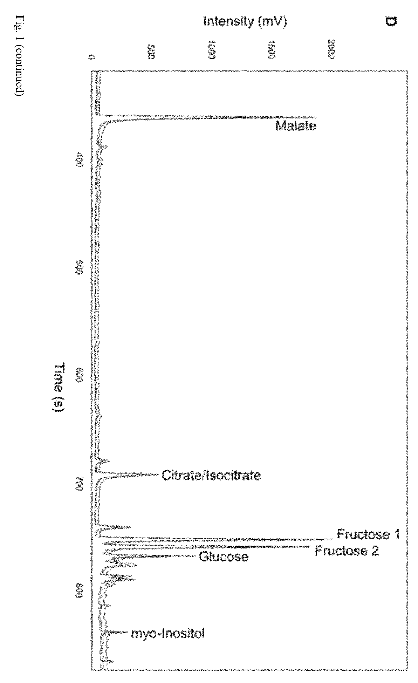

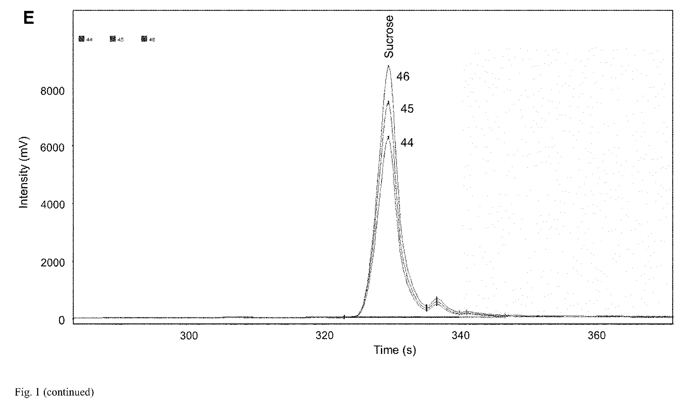

Fig. 1: GC-C-IRMS chromatogram of free amino acids and soluble sugars/organic acids: Chromatogram of free amino acids in shoot (A) and root (B) and soluble sugars/organic acids in shoot (C, E) and root (D) of a 15-day old, soil grown rice seedling at time point zero. The established protocol for metabolite extraction, derivatization and measurement by GC-C-IRMS yields good baseline separation for most metabolites, as well as a high signal to noise ratio. Each subfigure shows an overlay of the 3 analyzed mass traces 44 ($^{12}C^{16}O^{16}O$), 45 ($^{13}C^{16}O^{16}O$ and $^{12}C^{16}C^{17}O$) and 46 ($^{12}C^{18}O^{16}O$, $^{12}C^{17}O^{17}O$, $^{13}C^{17}O^{16}O$) for $CO_2$.

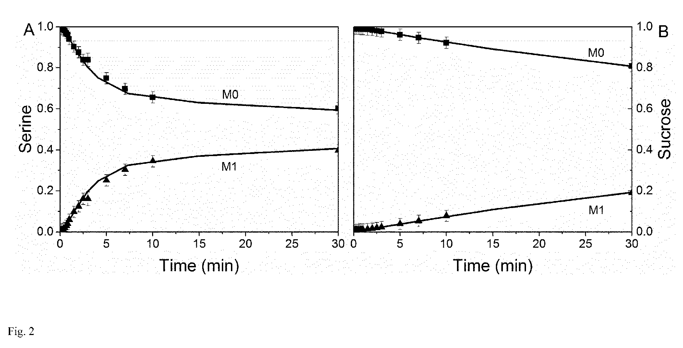

Fig. 2: Transient $^{13}C$ labeling profile of selected intracellular metabolites determined by GC-C-IRMS (serine (A), sucrose (B)). Serine shows fast label incorporation whereas sucrose is labeled more slowly. Data points show mass isotopomer data corrected for natural isotope abundance as mean values $\pm$ SD (n = 5). Soil-grown rice seedlings (15 days) were labeled with 400 $\mu$L L$^{-1}$ $^{13}CO_2$ for the following time periods: 0, 10, 20, 30, 40, 50, 60, 90, 120, 150, 180, 300, 420, 600 and 1800 seconds. (M0-M1 - mass isotopomers).

The following Examples shall merely illustrate the invention. They shall not be construed, whatsoever, to limit the scope of the invention.

**Example 1: Labeling study of rice seedlings**

Seed material of *Oryza sativa* L. ssp. *japonica* Nipponbare was obtained from CropDesign N.V. (Zwijnaarde, Belgium). Rice seeds were subjected to hot water treatment (60 °C, 10 min) and germinated on moist filter paper in a Petri dish for four days at 26 °C in the dark. Seeds were then transferred into light (500 $\mu$mol m$^{-2}$ s$^{-1}$ photosynthetically active radiation (PAR)), one day before they were transplanted into 0.7 dm3 pots used for further cultivation on soil (Einheitserde Type-GS90, 70% organic fiber peat, 30% clay, pH 5.5-6, Einheitserde-und Humuswerke Gebr. Patzer, Altengronau, Germany). Prior to the transfer, pots were soaked with deionized water, containing 0.15% of the fungicide proplant (Stähler, Stade, Germany). Rice plants were grown for 15 days under 13/11 h day/night cycles at an average irradiance of 500 $\mu$mol m$^{-2}$s$^{-1}$ PAR during the light phase (Powerstar HQI-BT 400W, Osram, Munich, Germany), temperature cycles of 26/21 °C and a relative humidity of 60%.

A sophisticated, gas-tight and largely automated flux incubator (0.75 x 0.75 x 1.1 m, 620 L volume) (disclosed in WO2014/079696) allowed the incubation of seedlings, as well as adult plants for individual or combined $^{13}CO_2$ and $^{15}NH_4NO_3$ labeling experiments.

Prior to labeling, 15 soil-grown rice seedlings were placed in a labeling chamber equipped with a sampling port allowing the fast harvest of individual plants without disturbing the reactor atmosphere. The air inside the chamber was pumped through soda lime pellets (Drägersorb® 800+, Mercateo, München, Germany) resulting in $CO_2$ depleted air being returned into the incubator. Immediately after adsorption of atmospheric $^{12}CO_2$, $^{13}CO_2$ was injected into the chamber to reach a concentration of 400 $\mu$L L$^{-1}$. During the experiment 15 plant samples were collected over a 30 minute interval at the following time points: 0, 10, 20, 30, 40, 50, 60, 90, 120, 150, 180, 300, 420, 600, 1800 seconds. The first sample time point was sampled directly after injection of $^{13}CO_2$ (corresponding control sample). The entire shoot was harvested and immediately quenched in liquid nitrogen. The sampling process was performed in one to two seconds. Five replicate experiments were conducted.

**Example 3: GC-C-IRMS for isotopic enrichment detection in free amino acids and sugars**

The isotopic enrichment of free amino acids, soluble sugars and malate was measured by GC-C-IRMS. Metabolite extraction was carried out, using 5 mg lyophilized and ground plant material, extracted with cold (-80°C) methanol/water/formic acid (80/20/1 (v/v/v)) using a ball mill (30 seconds, 30 Hz, Retsch MM300, Retsch, Haan, Germany). After centrifugation (13,000 xg, 10 min, 4 °C) the supernatant was subjected to solid phase extraction (Oasis MCX, Waters, Eschborn, Germany). For doing so, the supernatant was loaded onto the conditioned and equilibrated sorbent material and washed with the extraction solution (methanol/water/formic acid). The eluate, containing free sugars and organic acids, was collected and dried down. The dried residue was redissolved in 50 $\mu$L methoxyamine hydrochloride in pyridine (20 mg mL$^{-1}$) and derivatized for 90 minutes at 60 °C, followed by a second derivatization step (50 $\mu$L MSTFA, 30 minutes, 60 °C) (Roessner et al., 2000). Amino acids, bound to the solid phase, were subsequently eluted with 5% ammoniumhydroxid in acetonitril, dried down and derivatized for 60 minutes at 60 °C in pyridin and MBDSTFA (Macherey-Nagel, Düren, Germany). The protocol for labeling measurements by GC-C-IRMS (Trace GC Ultra Gas Chromatograph, Delta V Plus Isotope Ratio MS, Thermo Scientific, Waltham, USA-MA) was as follows: a defined sample volume was injected via the PTV-inlet (250 °C) at a split ratio of 1:20. Helium was used as carrier gas at a constant flow rate of 1 mL min$^{-1}$. Analytes were separated on a fused silica capillary column (HP-5MS, 30 m x 25 mm, 0.25 $\mu$m, Agilent, Waldbronn, Germany) and then transferred to an oxidizing combustion reactor (1000 °C). For analysis of free amino acids a sample volume of 1 $\mu$L was injected and separated with the following temperature program: 2 min at 100 °C, 10 °C min$^{-1}$ to 280 °C, 50 °C min$^{-1}$ to 340 °C and 1 min at 340 °C. For analysis of soluble sugars: 0.5 $\mu$L of sample was injected and separated with a temperature gradient: 1 min at 100 °C, 50 °C min$^{-1}$ to 140 °C, 5 °C min$^{-1}$ to 190 °C, 40 °C min$^{-1}$ to 340 °C and 2 min at 340 °C. For analysis of sucrose: 0.5 $\mu$L injection volume, 2

min at 200 °C, 50 °C min$^{-1}$ to 340 °C and 2 min at 340°C. Five biological replicates were analyzed.

The GC-C-IRMS profiles were obtained in selective ion monitoring (SIM) mode via the mass isomers of formed $CO_2$ (m/z 44, 45, 46) at their corresponding retention time, respectively. Metabolite identification was achieved independently by GC-MS (7890A GC-System, 7000 GC/MS Triple Quad, 7693 Autosampler, Agilent Technologies, Waldbronn, Germany) measurements of single amino acids and their mixture (10 $\mu$M each) in full-scan acquisition mode, followed by a NIST mass spectral search (NIST MS search 2.0) and comparison of the chromatographic pattern with the one of the corresponding GC-C-IRMS measurement. Data acquisition and evaluation was conducted using the Software Isodat NT (Thermo Scien-tific, Waltham, USA-MA).

Typical GC-C-IRMS chromatograms are shown in Fig. 1, Label incorporation in Fig 2.

## Claims

1. A method for extracting at least one isotope-labelled compound from a complex matrix sample comprising

   a) contacting said complex matrix sample with an extraction solvent comprising a water-miscible organic solvent and water
   b) binding cationic isotope-labelled compounds to a solid adsorbent in the presence of an acid,
   c) collecting non-cationic isotope-labelled compounds not binding to said solid adsorbent and/or eluting said cationic isotope-labelled compounds from said solid sorbent,
   d) thereby extracting at least one isotope-labelled compound from said complex matrix sample.

2. The method of claim 1, wherein said cationic isotope-labelled compound is an amino acid

3. The method of claim 1 or 2, wherein said non-cationic isotope-labelled compound is a saccharide and/or an organic acid.

4. The method of any one of claims 1 to 3, wherein said water-miscible organic solvent is an alcohol comprising 2 or 1 carbon atom(s), preferably is methanol, and/or wherein said water-miscible organic solvent is present at a concentration of from 10 % (v/v) to 95 % (v/v).

5. The method of any one of claims 1 to 4, wherein said extraction solvent further comprises an acid, preferably formic acid

6. The method of any one of claims 1 to 5, wherein said extraction solvent has a pH of at most 3, preferably of from 1 to 3, more preferably of about 2.5, most preferably of 2.5.

7. The method of any one of claims 1 to 6, wherein said solid adsorbent is a solid cation adsorbent.

8. The method of any one of claims 1 to 7, wherein said complex matrix sample is a biological matrix sample.

9. A method for determining at least one isotope-labelled compound comprised in a complex matrix sample, comprising

   a) contacting said complex matrix sample with an extraction solvent comprising a water-miscible organic solvent and water
   b) binding cationic isotope-labelled compounds to a solid adsorbent in the presence of an acid,
   c) collecting non-cationic isotope-labelled compounds not binding to said solid adsorbent and/or eluting said cationic isotope-labelled compounds from said solid sorbent,
   d) separating cationic isotope-labelled compounds by chromatographic steps and/or separating non-cationic isotope-labelled compounds by chromatographic steps, and
   e) determining, preferably quantitatively determining, cationic and/or non-cationic isotope-labelled compounds, thereby
   f) determining at least one isotope-labelled compound comprised in a complex matrix sample.

10. The method of claim 9, wherein said determining cationic isotope-labelled compounds by chromatographic steps and/or separating non-cationic isotope-labelled compounds comprises a non-compound discriminating determining step, preferably wherein said non-compound discriminating determining step is the only step of quantifying cationic and/or non-cationic isotope-labelled compounds.

11. The method of claim 9 or 10, wherein said non-compound discriminating determining step is and/or comprises mass spectrometry.

12. The method of any one of claims 9 to 11, wherein said non-compound discriminating determining step comprises, preferably is, isotope ratio mass spectrometry (IRMS), preferably is thermal conversion IRMS (TC-IRMS) of $^{18}$O-comprising compounds and/or of 2H-comprising compounds and/or is combustion (C-IRMS) of $^{13}$C-comprising compounds and/or of $^{15}$N-comprising compounds.

13. A kit comprising an extraction solvent as specified in any one of claims 1 to 6 and at least one isotope-labeled standard.

**14.** Use of a method according to any one of claims 1 to 12 in metabolic flux analysis.

**15.** Use of a solid adsorbent and/or of an extraction solvent comprising a water-miscible organic solvent and water in a method according to any one of claims 1 to 12.

Fig. 1A

Fig. 1 (continued)

Fig.                    1                    (continued)

Fig. 1 (continued)

Fig. 1 (continued)

Fig. 2

Europäisches
Patentamt

European
Patent Office

Office européen
des brevets

**EUROPEAN SEARCH REPORT**

Application Number

EP 16 18 3106

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHEN W P ET AL: "Microscale analysis of amino acids using gas chromatography-mass spectrometry after methyl chloroformate derivatization", JOURNAL OF CHROMATOGRAPHY B: BIOMEDICAL SCIENCES & APPLICATIONS, ELSEVIER, AMSTERDAM, NL, vol. 878, no. 24, 15 August 2010 (2010-08-15), pages 2199-2208, XP027193272, ISSN: 1570-0232 [retrieved on 2010-07-01] * whole document, in particular abstract; p. 2199, col. 1, bridging par. - col. 2, par. 1; p. 2199, col. 2 - p. 2200, col. 1, bridging par.; p. 2200, col. 2, par. 1-3; p. 2201, col. 1, par. 1 - p. 2202, col. 1, bridging par. * ----- | 1-15 | INV. G01N33/68 B01D59/26 |
| X | KAI-TING FAN ET AL: "Proteome Scale-Protein Turnover Analysis Using High Resolution Mass Spectrometric Data from Stable-Isotope Labeled Plants", JOURNAL OF PROTEOME RESEARCH., vol. 15, no. 3, 4 March 2016 (2016-03-04), pages 851-867, XP055342781, US ISSN: 1535-3893, DOI: 10.1021/acs.jproteome.5b00772 * whole document, in particular p. 854, col. 1, par. 1; p. 854, col. 2, par. 1; p. 855, col. 1, par. 1 - p. 857, col. 1, par. 1; p. 865, col. 1, par. 1 * ----- | 1,4-6, 8-15 | TECHNICAL FIELDS SEARCHED (IPC)  G01N B01D |

-/--

The present search report has been drawn up for all claims

| Place of search | Date of completion of the search | Examiner |
|---|---|---|
| The Hague | 7 February 2017 | Chrétien, Eva Maria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

**EUROPEAN SEARCH REPORT**

Application Number

EP 16 18 3106

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ATUL K SINGH ET AL: "Metabolomes of the psychrotolerant bacterium10403S grown at 37Â DEG C and 8Â DEG C", INTERNATIONAL JOURNAL OF FOOD MICROBIOLOGY, ELSEVIER BV, NL, vol. 148, no. 2, 10 May 2011 (2011-05-10), pages 107-114, XP028230840, ISSN: 0168-1605, DOI: 10.1016/J.IJFOODMICRO.2011.05.008 [retrieved on 2011-05-18] * whole document, in particular p. 108, col. 1, bridging par. - p. 109, col. 1, bridging par * | 1-15 | |
| Y | HEINZLE E ET AL: "Analysis of $^{13}C$ labeling enrichment in microbial culture applying metabolic tracer experiments using gas chromatography-combustion-isotope ratio mass spectrometry", ANALYTICAL BIOCHEMISTRY, ELSEVIER, AMSTERDAM, NL, vol. 380, no. 2, 15 September 2008 (2008-09-15), pages 202-210, XP023183007, ISSN: 0003-2697, DOI: 10.1016/J.AB.2008.05.039 [retrieved on 2008-06-17] * whole document, in particular abstract, p. 203, col. 1, bridging par. - p. 204, col. 1, bridging par. * | 1-15 | TECHNICAL FIELDS SEARCHED (IPC) |
| Y,D | WO 2014/079696 A1 (BASF PLANT SCIENCE CO GMBH [DE]) 30 May 2014 (2014-05-30) * whole document, in particular examples 1-3 * | 1-15 | |

-/--

The present search report has been drawn up for all claims

| Place of search | Date of completion of the search | Examiner |
|---|---|---|
| The Hague | 7 February 2017 | Chrétien, Eva Maria |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 
& : member of the same patent family, corresponding
    document

EPO FORM 1503 03.82 (P04C01)

Europäisches
Patentamt

European
Patent Office

Office européen
des brevets

**EUROPEAN SEARCH REPORT**

Application Number

EP 16 18 3106

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | IWAKOSHI E ET AL: "ISOLATION AND CHARACTERIZATION OF A GNRH-LIKE PEPTIDE FROM OCTOPUS VULGARIS", INTERNATIONAL CONGRESS OF COMPARATIVE ENDOCRINOLOGY, XX, XX, 1 January 2001 (2001-01-01), pages 585-590, XP001207729, * the whole document * | 1-15 | |

-----

TECHNICAL FIELDS
SEARCHED      (IPC)

The present search report has been drawn up for all claims

| Place of search | Date of completion of the search | Examiner |
|---|---|---|
| The Hague | 7 February 2017 | Chrétien, Eva Maria |

EPO FORM 1503 03.82 (P04C01)

page 3 of 3

**ANNEX TO THE EUROPEAN SEARCH REPORT
ON EUROPEAN PATENT APPLICATION NO.**

EP 16 18 3106

This annex lists the patent family members relating to the patent documents cited in the above-mentioned European search report.
The members are as contained in the European Patent Office EDP file on
The European Patent Office is in no way liable for these particulars which are merely given for the purpose of information.

07-02-2017

| Patent document cited in search report | Publication date | Patent family member(s) | Publication date |
|---|---|---|---|
| WO 2014079696 A1 | 30-05-2014 | AU 2013349905 A1 | 02-04-2015 |
| | | CA 2886211 A1 | 30-05-2014 |
| | | EP 2922947 A1 | 30-09-2015 |
| | | US 2015309038 A1 | 29-10-2015 |
| | | WO 2014079696 A1 | 30-05-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014079696 A

**Non-patent literature cited in the description**

- **HEINZLE et al.** *Analytical Biochemistry,* 2008, vol. 380, 202-210
- **CHEN et al.** *J. Chromatogr. B,* 2010, vol. 878, 2199
- **KASPAR et al.** *J. Chromatogr. B,* 2008, vol. 870, 222
- **MOLERO et al.** *Functional Plant Biology,* 2014, vol. 41, 331-341
- **MOLERO et al.** *Rapid Commun. Mass Spectrom.,* 2011, vol. 25, 599-607
- **LARSEN et al.** *Ecology,* 2009, vol. 90 (12), 3526-3535
- **DERSCH et al.** *Plant Physiology,* 2016, vol. 171, 25-41
- **BRENNA et al.** *Mass Spec Rev,* 1997, vol. 16, 227
- Isotope ratio mass spectrometry technique to follow plant metabolism. **GHASHGHAIE ; TCHERKEZ.** Advances in Botanical Research. 2013, vol. 67
- *Pure Appl. Chem.,* 2002, vol. 74 (11), 2169